# EUROPÄISCHE PATENTANMELDUNG

(11) **EP 1 500 384 A2**
(43) Veröffentlichungstag der Anmeldung: **26.01.2005**
(21) Anmeldenummer: 04015594.7
(22) Anmeldetag: 02.07.2004
(51) Int. Cl.: A61F 13/512

(54) **Perforierter Film mit strukturierter Oberfläche**

(30) Priorität: 23.07.2003 DE 20311313 U
(71) Anmelder: RKW AG Rheinische Kunststoffwerke, 67547 Worms (DE)
(72) Erfinder: Waller, Paul, 83026 Rosenheim (DE); Bauer, Peter, 83512 Wasserburg (DE)
(74) Vertreter: Wagner, Jutta, Dr.

(57) **Zusammenfassung**

Die vorliegende Erfindung betrifft einen perforierten Film, in welchem die Perforationen als Kapillaren ausgebildet sind, wobei dessen Oberfläche durch eine bleibende mechanische Verformung strukturiert ist und die Strukturierung aus einem Abstand von 30 cm mit bloßem Auge sichtbar ist.

## Beschreibung

Die vorliegende Erfindung betrifft einen perforierten Film mit Kapillaren als Perforation.

Derartige Filme finden insbesondere Verwendung im Hygiene- und Medizinbereich, zum Beispiel in der Damenhygiene. Vorzugsweise werden Hygienefolien aus Polyolefinen hergestellt und besitzen ein Flächengewicht im Bereich von 5 bis 70 g/m², insbesondere zwischen 15 und 30 g/m².

An Folien für Hygieneprodukte, speziell in der Damenhygiene, werden bestimmte Anforderungen gestellt. Die beschriebenen Folien sollen einerseits durchlässig für Körperflüssigkeiten sein, andererseits durch ihre dreidimensionale Ausformung der Öffnungen (Kapillare) ein unter Druckbelastung mögliches "zurückfließen" der Körperflüssigkeit aus dem Saugkissen zur Oberfläche vermeiden (re-wet). Des weiteren soll die Oberfläche möglichst eine Struktur aufweisen, die dieser ein trockenes Gefühl und mattes Aussehen verleiht. Diese Struktur kann aus einer Art Prägung bestehen oder aber aus einem aufgebrachtem Fasergebilde. In zunehmendem Maße soll der Oberfläche ein weiches, textilartiges Aussehen verliehen werden.

Für eine gute Funktion in der Anwendung bei absorbierenden Artikeln sollten möglichst freie Durchgänge vorhanden sein, um Körperflüssigkeit schnell und frei zum dahinterliegenden Saugkern durchtreten zu lassen.

Aus der US 4,463,045 ist ein perforierter Film aus thermoplastischem Polymermaterial bekannt, bei dem die Perforationen als Kapillaren ausgebildet sind und bei dem die Oberfläche eine Mikrostruktur aufweist. Die Oberfläche solcher Filme hat jedoch ein wenig textilähnliches Aussehen.

Des weiteren ist z. B. aus US 5,549,777, DE 39 19 166, DE 35 21 374 bekannt, die Oberfläche des perforierten Films mit einem Nonwoven zu bedecken. Dadurch wird zwar ein textilartiges Erscheinungsbild erzeugt, der freie Flüssigkeitsdurchtritt durch die Öffnungen des Films ist jedoch gehemmt.

Weiterhin ist aus der WO 98/10726 bekannt, die textilartige, weiche Struktur bei einem Polymerfilm durch ein spezielles Verfahren herzustellen. Mittels Hochdruckwasserstrahlen werden verschiedene Öffnungen (Perforationen) in dem Polymerfilm erzeugt und dadurch die Oberfläche textilartig. Dieses Verfahren hat jedoch den Nachteil, dass die Herstellung eines solchen perforierten Filmes sehr kostenintensiv ist.

Der Erfindung lag daher die Aufgabe zugrunde, die bekannten Filme, Laminate und Verfahren zu deren Herstellung dahingehend weiterzubilden bzw. zu ergänzen, dass perforierte Filme mit entsprechender Oberfläche erhalten werden, bei denen die obengenannten Nachteile nicht oder nur in erheblich verringertem Umfang auftreten.

Zur Lösung der Aufgabe wird ein perforierter Film vorgeschlagen, der ausgebildete Kapillaren besitzt (male Seite) und dessen Oberfläche (female Seite) eine sekundäre Struktur (Makroverformung) aufweist, die eine optisch textilartige Oberfläche schafft.

### Ein derartiger Film hat folgende Vorteile:

Die Kapillaren sind gut ausgeformt, so dass ein Transport der Körperflüssigkeit von der Oberfläche weg zur gegenüberliegenden Seite in ein Saugkissen erfolgen kann, wobei durch die Form und Ausführung der Kapillare ein unter eventueller Druckbelastung stattfindendes Zurückfließen der Flüssigkeit ganz oder zumindest weitgehend verhindert wird.

Die Oberfläche der perforierten Folie ist bleibend mechanisch verformt und dadurch strukturiert. Die Strukturierung ist so zu bemessen, dass sie mit dem bloßen Auge aus einem Abstand von 30 cm sichtbar ist. Hierdurch entsteht eine matte, sich weich, trocken und angenehm anfühlende, textilartige Oberfläche.

Die Strukturierung besteht vorzugsweise aus Erhebungen oder Vertiefungen, welche einen Abstand von 1 bis 6 mm voneinander aufweisen, d.h. dass die höchsten bzw. tiefsten Punkten jeweils etwa 1 bis 6 mm auseinander liegen.

Bei einer bevorzugten Ausführungsform ist die Oberfläche wellenartig strukturiert. Durch die in Längswellen angeordneten Verformungen entsteht eine Struktur, die einem textilen Feinrippgewebe optisch ähnlich sieht. Ein weiterer Vorteil einer solchen wellenartigen Struktur ist, dass beim Aufbringen von Flüssigkeit diese nicht quer zu den Verformungen fließen kann, bevor sie durch die Öffnungen der Folie tritt. Dieser Effekt hat Vorteile beim Einsatz einer derartigen Folie in der Damenhygiene, da Körperflüssigkeit nicht seitlich abfließen kann, bevor sie durch die Perforationen tritt und vom darunter liegendem Saugkissen aufgenommen wird. Dadurch kann eine seitliche Leckage deutlich reduziert werden.

Die Herstellung einer derartig verformten perforierten Folie erfolgt in einem Arbeitsschritt, kann aber, falls die Gegebenheiten es wünschenswert erscheinen lassen, auch in 2 oder mehreren Arbeitsschritten erfolgen. Zur Herstellung der erfindungsgemäßen Filme dient vorzugsweise eine Unterdruck-Perforierungsvorrichtung, ausgestattet mit einem perforierten Zylinder oder Band mit einem darunter befindlichem Vakuumsegment und entsprechenden Heiz- und Kühlvorrichtungen, mittels derer entweder ein fertiger Film erhitzt und im schmelzeflüssigen Zustand perforiert wird oder mittels derer ein direkt aus einer Breitschlitzdüse mit vorgeschalteten Extruder kommender schmelzeflüssiger Polymerfilm perforiert wird.

Bekanntlich besitzen Polymere keinen scharf definierten Schmelzpunkt, sondern einen Schmelzbereich, wobei sich jedoch kristallinen Bereichen des Polymermaterials jeweils ein Kristallitschmelzpunkt zuordnen lässt. Dieser Kristallitschmelzpunkt liegt stets höher als der Schmelzpunkt (Schmelzbereich) der nicht kristallinen Bestandteile. In jedem Fall ist der schmelzeflüssige Zustand dadurch beschrieben, dass der Schubmodul gegen Null geht - und im Falle von Polymeren mit kristallinen Bereichen - letztere nicht mehr nachweisbar sind.

Die bleibende mechanische Verformung kann beispielsweise durch Erwärmen des Films und Einpressen der gewünschten Struktur erfolgen. Bevorzugt wird die bleibende mechanische Verformung durch entsprechend strukturierte Walzen erzeugt. Nachdem die fertig perforierte Folie gekühlt wurde, wird sie daher bevorzugt einem Walzenpaar zugeführt, wobei die Walzen mit der gewünschten Struktur versehen sind und die Erhebungen der ersten Walze in die Vertiefungen der zweiten Walze eingreifen. Die Walzenpaare können sowohl Raumtemperatur haben, als auch temperiert sein. Die Temperatur der Walze muss jedoch niedriger sein als der Beginn des Schmelzbereichs des Polymers. Die Struktur wird dadurch erzeugt, dass die Erhebungen der einen Walze in die Vertiefungen der zweiten Walze eingreifen, wodurch die dazwischen liegende bzw. laufende Folie entsprechend verformt wird.

Die Form der aufgebrachten Struktur unterliegt prinzipiell keiner Beschränkung, vorzugsweise besteht diese Struktur aus radialen Rillen oder Wellen. Die Höhe bzw. Tiefe der Struktur bzw. der Rillen und deren Abstand zueinander, hängt ab von der gewünschten Verformung der Folie. Die Eintauchtiefe der Walzen sollte jedoch zwischen 1 mm und 5 mm, bevorzugt zwischen 2 mm und 4 mm betragen. Der Abstand der Struktur bzw. der Rillen soll zwischen 1 mm und 6 mm, bevorzugt zwischen 2 mm und 4 mm betragen.

Alternativ können auch andere Strukturen wie z.B. rundliche oder eckige oder unregelmäßig geformte Erhebungen und/oder Einsenkungen vorgesehen sein.

Überraschenderweise wurde festgestellt, dass die perforierte Folie durch die Struktur der Walzen im kalten Zustand so verformt wird, so dass in der Folie eine textilartige Gesamtstruktur (textilartiges Aussehen) entsteht.

In einer bevorzugten Ausführungsform betrifft die Erfindung ein strukturiertes Foliengebilde aus einem perforierten Film aus Polymermaterial, wobei die Perforationen als Kapillaren ausgebildet sind und dessen Oberfläche mechanisch dahingehend bleibend verformt wurde, dass eine wellenartige Struktur entsteht.

Je nach Anwendungsgebiet und Zielsetzung kann die wellenartige Strukturierung sowohl quer als auch längs oder quer und längs zu der perforierten Folienbahn verlaufen. In einem bevorzugten Fall verläuft sie quer zur Folienbahn.

Als Polymermaterial für die Herstellung des erfindungsgemäßen, strukturierten Filmes sind alle zu schmelzbaren Polymerfilmen verarbeitbaren Polymermaterialien geeignet, wobei der Fachmann aus allen bekannten Materialien mit dieser Eigenschaft leicht die geeigneten herausfinden kann.

Bevorzugt sind Polyolefine oder Polyolefin-Copolymere, insbesondere aus Polyethylen, Polyethylen-Copolymer, Polypropylen oder Polypropylen-Copolymer, wobei Mischungen aus Polyethylen und Polyethylen-Copolymeren (LDPE (Low Density Polyethylene), MDPE (Medium Density Polyethylene) und LLDPE (Linear Low Density Polyethylene)) besonders bevorzugt sind.

In einer anderen Ausführungsform werden biologisch abbaubare Polymermaterialien eingesetzt, welche gemeinsam mit einem Saugkern aus Zellstoff kompostierbare Hygieneprodukte ergeben.

Üblicherweise werden die erfindungsgemäßen Folien gemäß ihrem Einsatzzweck in der Regel mit Titandioxid weiß eingefärbt. Weiterhin können die erfindungsgemäßen Foliengebilde die dem Fachmann bekannten Zusatzstoffe und / oder Verarbeitungshilfsmittel enthalten.

In einer besonders bevorzugten Ausführungsform der Erfindung enthält das Polymermaterial in Abhängigkeit von den Anforderungen des Anwendungsbereiches zusätzlich ein oberflächenaktives Additiv, das je nach Anforderung z. B. aus einem Hydrophobiermittel oder einem Hydrophiliermittel besteht.

Der perforierte Film kann aus zwei oder mehreren koextrudierten Schichten bestehen. In einer bevorzugten Ausführungsform der Erfindung besteht der Polymerfilm aus einer einzigen Schicht.

Die Schichtdicke des perforierten und verformten Filmes ist abhängig vom Flächengewicht des Films, von der Länge der Kapillaren, der Rezeptur des Ausgangsfilmes und der Höhe der Verformung. Vorzugsweise liegt die Gesamtdicke im Bereich von 0,5 bis 1,5 mm. Das Flächengewicht des perforierten Gebildes liegt vorzugsweise im Bereich von 15 bis 30 g/m².

Die Lochform des Perforierwerkzeuges und die daraus resultierende Form der Öffnungen unterliegen an sich keiner Beschränkung. Sie können sowohl eine runde als auch eine eckige Form aufweisen. Die Wände der Kapillare können parallel zueinander verlaufen, d.h. der mittlere Durchmesser der Kapillare ist an jeder Stelle der Kapillare gleich. Die Wände der Kapillare können aber auch ausgehend von den Eingangsöffnungen der Kapillare in Richtung auf die Ausgangsöffnungen konisch verlaufen, so dass der mittlere Durchmesser der Kapillare nicht konstant ist. Bei einer runden Form beträgt der Durchmesser der Eingangsöffnungen der Kapillare vorzugsweise 0,3 bis 1,0 mm, insbesondere 0,5 bis 0,8 mm. Die Kapillaren können sowohl senkrecht zur Oberfläche d.h. in einem Winkel von 90° zur Oberfläche, als auch in einem Winkel ungleich 90° d.h. schräg zur Oberfläche angeordnet sein.

Die Anzahl und Größe der Öffnungen ist abhängig von der geforderten Durchlässigkeitsmenge. Vorteilhafterweise sollte die offene Fläche der perforierten Folie 5 bis 40 %, vorzugsweise 20 bis 35 %, bezogen auf die Gesamtoberfläche der Folie betragen.

### Beispiel:

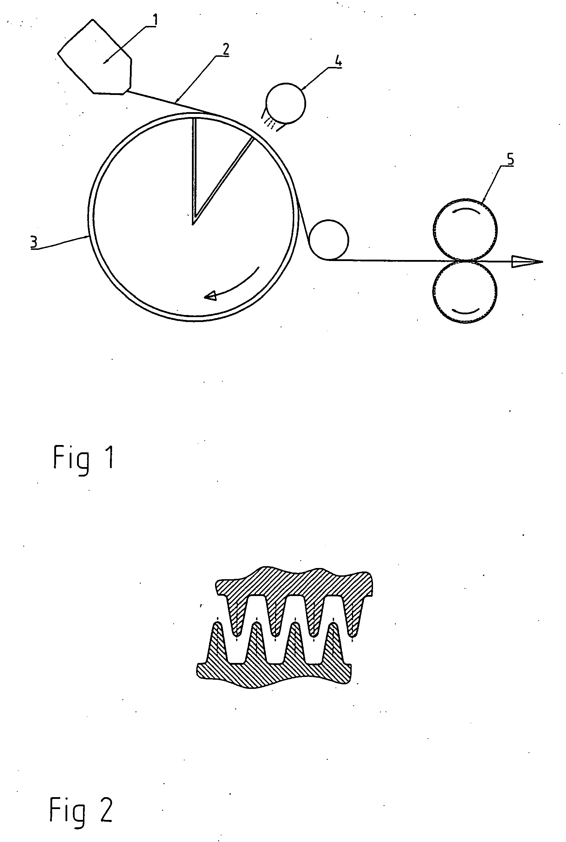

Auf der in Figur 1 schematisch dargestellten Anlage wurde aus einer Breitschlitzdüse 1 eine Ausgangsfolienbahn 2 aus 90 Gew.-Teilen LDPE (Lupolen 2420), 7 Gew.-Teilen Titandioxid Batch (Novachrome RB 6 AE) und 3 Gew.-Teilen Hydrophiliermittel-Batch (Polybatch AF 1088) mit einer Schmelzetemperatur von 260° C einem Perforierzylinder 3 zugeführt, wobei der Polymerfilm ein Flächengewicht von 25 g/m² hatte. Die Anzahl der Öffnungen pro cm² Zylinderoberfläche betrug 44, wobei die Bohrungen des perforierten Zylinders unter einem Winkel von 90° C angeordnet waren. Im Inneren des Zylinders befindet sich ein Vakuumsegment. Durch die Druckdifferenz erfolgt eine dreidimensionale Perforation der Folienschmelze, die anschließend mittels Kühlluft aus einem Gebläse 4 gekühlt wird.

Die erhaltene perforierte Folie wurde dann einem Walzenpaar 5 mit radialen Rillen, wie in Figur 2 schematisch dargestellt zugeführt. Der Abstand der Rillen betrug 2,8 mm, die Eintauchtiefe betrug 2,6 mm. Die perforierte Folie wurde beim Durchlauf des Walzenpaares in einer Weise bleibend verformt, dass diese ein textilartiges Aussehen annahm.

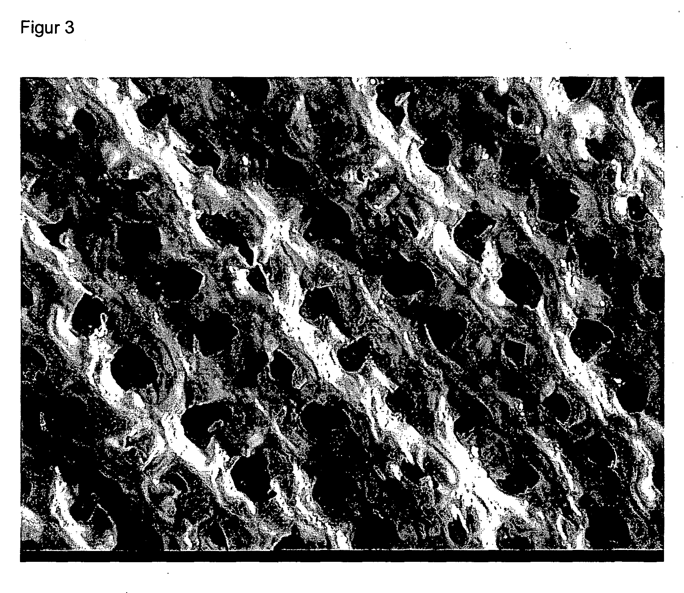

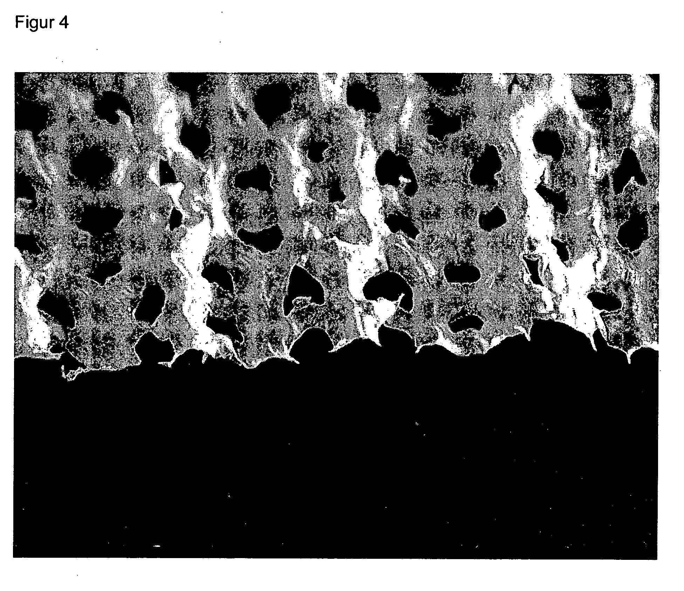

Figur 3 und Figur 4 zeigen lichtmikroskopische Aufnahmen einer dadurch erhaltenen textilartigen perforierten Folie.

## Patentansprüche

1. Perforierter Film, wobei die Perforationen als Kapillaren ausgebildet sind, **dadurch gekennzeichnet, dass** dessen Oberfläche durch eine bleibende mechanische Verformung strukturiert ist und die Strukturierung aus einem Abstand von 30 cm mit bloßem Auge sichtbar ist.

2. Film gemäß Anspruch 1, **dadurch gekennzeichnet, dass** die Strukturierung aus Erhebungen oder Vertiefungen mit einem Abstand im Bereich von 1 mm bis 6 mm voneinander besteht

3. Film gemäß Anspruch 1 oder 2, **dadurch gekennzeichnet, dass** die Strukturierung aus Rillen besteht.

4. Film gemäß einem der Ansprüche 1 bis 3, **dadurch gekennzeichnet, dass** der Film aus Polymermaterial besteht.

5. Film gemäß Anspruch 4, **dadurch gekennzeichnet, dass** das Polymermaterial ein LDPE, MDPE, LLDPE, Polypropylen oder ein Gemisch davon ist.

6. Film gemäß Anspruch 4, **dadurch gekennzeichnet, dass** das Polymermaterial ein biologisch abbaubarer Stoff ist.

7. Film gemäß einem der Ansprüche 4 bis 6, **dadurch gekennzeichnet, dass** das Polymermaterial ein oberflächenaktives Additiv enthält.

8. Film gemäß einem der vorhergehenden Ansprüche, **dadurch gekennzeichnet, dass** die offene Fläche des perforierten Filmes 5 bis 40 %, vorzugsweise 20 bis 35 %, bezogen auf die Gesamtoberfläche des Films beträgt.

9. Film gemäß einem der vorhergehenden Ansprüche, **dadurch gekennzeichnet, dass** die bleibende mechanische Verformung als wellenartige Struktur ausgebildet ist.

10. Film gemäß einem der vorhergehenden Ansprüche, **dadurch gekennzeichnet, dass** die wellenartige Struktur in Längsrichtung zur Folienbahn des Films ausgebildet ist.

11. Film gemäß einem der vorhergehenden Ansprüche, **dadurch gekennzeichnet, dass** die wellenartige Struktur in Querrichtung zur Folienbahn des Films ausgebildet ist.

12. Film gemäß einem der vorhergehenden Ansprüche, **dadurch gekennzeichnet, dass** die wellenartige Struktur in einem beliebigem Winkel zur Folienbahn des Films ausgebildet ist.

13. Film gemäß einem der vorhergehenden Ansprüche, **dadurch gekennzeichnet, dass** die wellenartige Struktur sowohl längs als auch quer oder in einem beliebigem Winkel zur Folienbahn des Films ausgebildet ist.
